# EUROPEAN PATENT SPECIFICATION

(11) **EP 3 583 426 B1**
(45) Date of publication and mention of the grant of the patent: **28.06.2023**
(21) Application number: 18713032.3
(22) Date of filing: 16.02.2018
(51) Int. Cl.: G01N 33/574

(54) **METHODS FOR PREDICTING OVERALL AND PROGRESSION FREE SURVIVAL IN SUBJECTS HAVING CANCER USING CIRCULATING CANCER ASSOCIATED MACROPHAGE-LIKE CELLS (CAMLS)**
VERFAHREN ZUR VORHERSAGE DES GESAMT- UND DES PROGRESSIONSFREIEN ÜBERLEBENS BEI PATIENTEN MIT KREBS UNTER VERWENDUNG VON ZIRKULIERENDEN KREBSASSOZIIERTEN MAKROPHAGENARTGEN ZELLEN (CAMLS)
PROCÉDÉS DE PRÉDICTION DE LA SURVIE GLOBALE ET SANS PROGRESSION CHEZ DES SUJETS ATTEINTS D'UN CANCER, À L'AIDE DE CELLULES CIRCULANTES DE TYPE MACROPHAGE ASSOCIÉES AU CANCER (CTMAC)

(30) Priority: 16.02.2017 US 201762459961 P; 31.03.2017 US 201762479664 P; 08.05.2017 US 201762503041 P; 18.01.2018 US 201862618728 P
(43) Date of publication of application: 25.12.2019
(73) Proprietor: Creatv Microtech, Inc., Potomac, MD 20854 (US)
(72) Inventor: ADAMS, Daniel, Basking Ridge, NJ 07920 (US); TANG, Cha-Mei, Potomac, MD 20854 (US)
(74) Representative: Ullrich & Naumann PartG mbB
(86) International application number: PCT/US2018/000082
(87) International publication number: WO 2018/151865

(56) References cited:
- WO-A1-2013/181532
- WO-A1-2016/033103
- WO-A2-2010/005991
- US-A1- 2014 315 295
- US-A1- 2016 178 630

## Description

### BACKGROUND

### Field of the Invention

The present invention generally relates to the use of biomarkers in the blood and other bodily fluids to make predictions regarding overall survival and progression free survival in subjects having cancer, such as solid tumors.

### Related Art

When tumor cells break away from primary solid tumors, they penetrate into the blood or lymphatic circulation, and ultimately leave the blood stream and enter either organs or tissue to form metastasis. 90% of cancer-related deaths are caused by the metastatic process. The most common metastatic sites are the lung, liver, bone and brain. Tumor cells found in the circulation are called circulating tumor cells (CTCs). Many research publications and clinical trials show that CTCs have clinical utility in (i) providing prognostic survival and cancer recurrence information through the enumeration of CTCs in the blood stream, and (ii) providing treatment information through examination of protein expression levels, and the occurrence of gene mutations and translocations in the CTCs. However, CTCs are not consistently associated with the development and/or presence of cancer in a subject, even in stage IV cancer patients. While CTCs are found most often in stage IV of breast, prostate and colorectal cancers, they are rare in early stages of the same cancer. CTCs are also rare in other cancers.

Circulating cancer associated macrophage-like cells (CAMLs) are another cancer-related cell type that is found in the blood of subjects having cancer. CAMLs are associated with all solid tumors tested and all stages of cancer. CAMLs are polyploid and very large in size, ~25 µm to ~300 µm in size. These polyploid cells can be either CD45(-) or CD45(+) and express CD11c, CD 14 and CD31, which confirms their origin as a myeloid lineage. They are often found in the process of engulfing CTCs and cell debris [1,7].

Assays associated with the identification and characterization of biomarkers, such as CAMLs, in blood and other body fluids can be used to provide prognostic information. The present invention is directed to providing such tools to clinicians and other important goals.

US 2016/178630 A1 relates to a methodology to categorize circulating tumor cells (CTCs) into morphologically distinct subpopulations and to use one of the subpopulations in methods for predicting overall survival of a patient having cancer. WO 2016/033103 A1 is an earlier application of the present applicant and is directed to the discovery of "circulating Cancer Associated Macrophage-Like cell" (CAML) which are described in detail. Moreover, WO 2016/033103 A1 describes an association of the presence of CAMLs with solid tumors.
US 2014/315295 relates to microfilters having a hydrophilic surface that can be used to collect selected components such as cells from a fluid in particular bodily fluids like whole blood, urine or bone marrow. US 2014/315295 further describes devices comprising such microfilters as well as methods of using the microfilters in the removal or collection of cells from the fluid. Exemplary cells collected with the microfilters are CTCs and CAMLs. WO 2013/181532 A1 is a further application relating to the use of the presence of CAMLs as a biomarker of solid tumors in biofluids. In particular, the presence of CAML can be used to determine the presence of carcinomas, rapid determination of treatment response, or early detection of cancer.
WO 2010/005991 A2 relates to a method of detecting circular tumor cells (CTCs) and methods of detecting, evaluating or staging cancer in the patient. The detection method comprises contacting a sample of a patient with a CD45 binding agent labeled nucleic acid probes.

### SUMMARY

The present invention is directed methods of using a type of cell with unique characteristics that is found in the blood of subjects having solid tumors, including carcinoma, sarcoma, neuroblastoma and melanoma. These circulating cells, termed a "circulating Cancer Associated Macrophage-like cell" (CAML), has been shown to be associated with the presence of solid tumors in a subject having cancer. Five morphologies associated with CAMLs have been characterized and described [1,2]. CAMLs have been found consistently in the peripheral blood of subjects having stage I to stage IV solid tumors by microfiltration using precision microfilters.

Medical applications associated with CAMLs include, but are not limited to, use of the cells as a biomarker to provide early detection of cancer and diagnosis of cancer, in particular, in the early detection and diagnosis of cancer relapse or recurrence, and in the determination of cancer mutation. CAMLs are also shown through the data presented herein to have clinical utility as a biomarker in making predictions regarding disease progression and patient survival.

CAMLS may be used independently as a cancer marker, or in combination with circulating tumor cells (CTCs), epithelial mesenchymal transition calls (EMTs), circulating cancer associated vascular endothelial cells (CAVEs), cell-free DNA (cfDNA), circulating tumor DNA (ctDNA), methylated DNA, proteomic, metabolomic, lipidomic and other biomarkers to provide a more complete understanding of a patient's disease. More specifically, and in a first embodiment, the present invention is directed to a method for predicting overall survival (OS) and progression free survival (PFS) of a subject having cancer, said method comprising determining the size of circulating cells in a biological sample from a first subject having cancer, and comparing the results with a second subject having cancer, wherein the OS and PFS of the subject with larger-sized cells is predicted to be less than the OS and PFS of the subject having smaller-sized cells, wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

In one aspect, the method for predicting OS and PFS of a subject having cancer, said method comprising determining the size of circulating cells in a biological sample from a subject having cancer, wherein when at least one cell in said sample is about 50 µm or more in size, the OS and PFS of the subject is predicted to be less than a subject having cancer where none of the cells is more than about 50 µm in size, wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

In a second embodiment, the present invention is directed to a method for predicting OS and PFS of a subject having cancer, said method comprising determining a ratio of circulating cells in a selected volume of a biological sample from a subject having cancer, wherein when said ratio is equivalent to about 6 or more cells per 7.5 mL of the biological sample, the OS and PFS of the subject is predicted to be less than a subject having cancer with a ratio equivalent to less than 6 cells per 7.5 mL of the biological sample wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous..

In a third embodiment, the present invention is directed to a method for predicting OS and PFS of a subject having cancer, said method comprising determining the number of CEP17 dots in circulating cells in a biological sample from a subject having cancer, wherein when at least one cell in said sample has about ten or more CEP17 dots, the OS and PFS of the subject is predicted to be less than a subject having cancer where none of the cells in a biological sample from the subject has about ten or more CEP17 dots, wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

In certain aspects of the embodiments of the invention, OS or PFS, or both, is over a period of at least 12 months. In other aspects of the embodiments of the invention, OS or PFS, or both, is over a period of at least 24 months.

In certain aspects of the embodiments of the invention, the size of the biological sample is between 5 and 15 mL.

In each of the embodiments and aspects of the invention, the circulating cells are defined as having each of the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

In certain aspects of the embodiments of the invention, the circulating cells can be further defined as having one or more of the following additional characteristics:
(d) CD14 positive phenotype;
(e) CD45 expression;
(f) EpCAM expression;
(g) vimentin expression;
(h) PD-L1 expression;
(i) monocytic CD11C marker expression;
(j) endothelial CD146 marker expression;
(k) endothelial CD202b marker expression; and
(l) endothelial CD31 marker expression.

In each aspect and embodiment of the invention, the circulating cells can be termed "Cancer Associated Macrophage-like cells" or CAMLs.

In certain aspects of the embodiments of the invention, the source of the biological sample may be, but is not limited to, one or more of peripheral blood, blood, lymph node, bone marrow, cerebral spinal fluid, tissue, and urine. When the biological sample is blood, the blood may be antecubital-vein blood, inferior-vena-cava blood, femoral vein blood, portal vein blood, or jugular-vein blood, for example. The sample may be a fresh sample or a properly prepared cryo-preserved sample that is thawed.

In certain aspects of the embodiments of the invention, the cancer is a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer.

In certain aspects of the embodiments of the invention, the circulating cells are isolated from the biological samples for the determining steps using one or more means selected from size exclusion methodology, immunocapture, red blood cell lysis, white blood cell depletion, FICOLL density gradient, electrophoresis, dielectrophoresis, flow cytometry, magnetic levitation, and various microfluidic chips, or a combination thereof.

In one aspect of the invention, circulating cells are isolated from the biological samples using size exclusion methodology that comprises using a microfilter. The microfilter may have a pore size ranging from about 5 microns to about 20 microns. The pores of the microfilter may have a round, race-track shape, oval, square and/or rectangular pore shape. The microfilter may have precision pore geometry and/or uniform pore distribution.

In another aspect of the invention, circulating cells are isolated from the biological samples using a microfluidic chip via physical size-based sorting, hydrodynamic size-based sorting, grouping, trapping, immunocapture, concentrating large cells, or eliminating small cells based on size.

In a further aspect of the invention, circulating cells are isolated from the biological samples using a CellSieve^{™} low-pressure microfiltration assay.

### BRIEF DESCRIPTION OF THE DRAWINGS

**Figure 1** shows the intensity of cell differentiation markers used to identify and subtype CAMLs.
**Figure 2** compares the presence of CTCs versus CAMLsin different stages of cancer.
**Figures 3A-3B** show the average number of CAMLs by size in different stages of cancer.
**Figure 4** shows the relationship between CAML size and the number of CEP17 dots per cell.
**Figure 5** shows plots of probability of overall survival (OS) for 24 months for a discovery set of n=157 patients and validation set of n=158 patients. The data is analyzed for CAML size ≥ 50 µm and < 50 µm. Discovery set (n=157); Hazard ratio: 3.4 (95%CI=2.1-5.6, p<0.001). Validation set (n=158); Hazard ratio: 4.2 (95%CI=2.3-7.7, p<0.001).
**Figure 6** shows plots of probability of progression free survival (PFS) for 24 months for a discovery set of n=157 patients and validation set of n=158 patients. The data is analyzed for CAML size ≥ 50 µm and < 50 µm. Discovery set (n=157); Hazard ratio: 3.2 (95%CI=2.1-5.0, p<0.001). Validation set (n=158); Hazard ratio: 3.8 (95%CI=2.7-6.8, p<0.001).
**Figure 7** shows OS (left panel) and PFS (right panel) for 24 months analyzed for n=293 patient samples for CAML number ≥ 6 and <6.
**Figure 8** shows OS (left panel) and PFS (right panel) for 24 months analyzed for n=293 patient samples for CAML size ≥ 50 µm and < 50 µm.
**Figure 9** shows PFS for 24 months analyzed for n=293 patient samples separated into Stages I and II (left panel) and for Stages III and IV (right panel) for CAML size < 50 µm, 50-110 µm and > 110 µm.
**Figure 10A** shows OS (left panel) and PFS (right panel) for Stage I patients (n=62) for 24 months analyzed for CAML size ≥ 50 µm and < 50 µm.
**Figure 10B** shows OS (left panel) and PFS (right panel) for Stage II patients (n=72) for 24 months analyzed for CAML size ≥ 50 µm and < 50 µm.
**Figure 10C** shows OS (left panel) and PFS (right panel) for Stage III patients (n=69) for 24 months analyzed for CAML size ≥ 50 µm and < 50 µm.
**Figure 10D** shows OS (left panel) and PFS (right panel) Stage IV patients (n=106) for 24 months analyzed for CAML size ≥ 50 µm and < 50 µm.
**Figure 11A** shows OS (left panel) and PFS (right panel) for breast cancer patients (n=59) for 24 months analyzed for CAML size ≥ 50 µm and < 50 µm.
**Figure 11B** shows OS (left panel) and PFS (right panel) for esophageal cancer patients (n=27) for 24 months analyzed for CAML size ≥ 50 µm and < 50 µm.
**Figure 11C** shows OS (left panel) and PFS (right panel) for non-small cell lung cancer (NSCLC) patients (n=59) for 24 months analyzed for CAML size ≥ 50 µm and < 50 µm.
**Figure 11D** shows OS (left panel) and PFS (right panel) for pancreatic cancer patients (n=59) for 24 months analyzed for CAML size ≥ 50 µm and < 50 µm.
**Figure 11E** shows OS (left panel) and PFS (right panel) for prostate cancer patients (n=74) for 24 months analyzed for CAML size ≥ 50 µm and < 50 µm.
**Figure 11F** shows OS (left panel) and PFS (right panel) for renal cell carcinoma (RCC) patients (n=37) for 24 months analyzed for CAML size ≥ 50 µm and < 50 µm.

### DETAILED DESCRIPTION

Cancer is one of the most feared illness in the world, affecting all populations and ethnicities in all countries. Approximately 40% of both men and women will develop cancer in their lifetime. In the United States alone, at any given time there are more than 12 million cancer patients, with 1.7 million new cancer cases and more than 0.6 million deaths estimated for 2018. Cancer death worldwide is estimated to be about 8 million annually, of which 3 million occur in developed countries where patients have access to treatment.

Ideally, there will be diagnostics that can quickly determine if a selected therapy is working and provide prognosis for survival.

In this disclosure, a cell type is presented that is more consistently found in the blood of solid tumor patients from Stages I-IV than any other cancer related cell. These circulating cells are macrophage-like cells that contain the same tumor markers as the primary tumor and they are termed circulating Cancer Associated Macrophage-like cells (CAMLs) herein.

Along with circulating tumor cells (CTCs), CAMLs present in biological samples from patients having cancer can be isolated and characterized, for example through the use of size exclusion methods, including microfiltration methods. Microfilters can be formed with pores large enough to allow red blood cells and the majority of white blood cells to pass, while retaining larger cells such as CTCs and CAMLs. The collected cells can then be characterized, either directly on the filters or through other means.

CAMLs have many clinical utilities when used alone. Furthermore, the characterization of CAMLs in a biological sample can be combined with the assaying of other markers such as CTCs, cell-free DNA and free proteins in blood to further improve sensitivity and specificity of a diagnosis technique. This is especially true for CAMLs and CTCs because they can be isolated and identified at the same time using the same means.

### Circulating Tumor Cells

As defined herein, CTCs associated with carcinomas express a number of cytokeratins (CKs). CK 8, 18, & 19 are the cytokeratins most commonly expressed and used in diagnostics, but surveying need not be limited to these markers alone. The surface of solid tumor CTCs usually express epithelial cell adhesion molecule (EpCAM). However, this expression is not uniform or consistent. CTCs do not express any CD45 because it is a white blood cell marker. In assays to identify tumor-associated cells, such as CTCs and CAMLs, it is sufficient to use antibodies against markers associated with the solid tumor such as CK 8, 18, & 19, or antibodies against CD45 or DAPI. Combining staining techniques with morphology, pathologically-definable CTCs (PDCTC), apoptotic CTCs and CAMLs can be identified [3].

PDCTCs associated with solid tumors express CK 8, 18, & 19, and can be identified and defined by the following characteristics:
- A "cancer-like" nuclei stained by DAPI. The nuclei are usually large with dot patterns. The exception is when the cell is in division. The nucleus can also be condensed.
- Expression of one or more of CK 8, 18 and 19; CTCs from epithelial cancers usually express at least CK 8, 18 and 19. The cytokeratins have a filamentous pattern.
- Lack of CD45 expression.

An apoptotic CTCs associated with cancer express CK 8, 18, & 19 and can be identified and defined by the following characteristics:
- A degrading nuclei.
- Expression of one or more of CK 8, 18 and 19; not all the cytokeratins are filamentous in pattern, but parts or whole appear fragmented in the form of spots.
- Lack of CD45 expression.

### Circulating Cancer Associated Macrophage-like Cells (CAMLs)

As defined herein, the circulating cells used in the methods of the invention can be termed "CAMLs". Each reference to "circulating cells" is synonymous with CAMLs, and each reference to "CAMLs" is synonymous with circulating cells. Whether termed CAMLs or "circulating cells" these cells are characterized by having one or more of the following features:
- CAMLs have a large, atypical polyploid nucleus or multiple individual nuclei, often scattered in the cell, though enlarged fused nucleoli are common. CAML nuclei generally range in size from about 10 µm to about 70 µm in diameter, more commonly from about 14 µm to about 64 µm in diameter.
- For many cancers, CAMLs express the cancer marker of the disease. For example, CAMLs associated with epithelial cancers may express CK 8, 18 or 19, vimentin, etc. The markers are typically diffused, or associated with vacuoles and/or ingested material. The staining pattern for any marker is nearly uniformly diffused throughout the whole cell. For sarcomas, neuroblastomas and melanomas, other markers associated with the cancers can be used instead of CK 8, 18, 19.
- CAMLs can be CD45 positive or CD45 negative, and the present invention encompasses the use of both types of CAMLs.
- CAMLs are large, approximately 20 micron to approximately 300 micron in size by the longest dimension.
- CAMLs are found in many distinct morphological shapes, including spindle, tadpole, round, oblong, two legs, more than two legs, thin legs, or amorphous shapes.
- CAMLs from carcinomas typically have diffused cytokeratins.
- If CAMLs express EpCAM, EpCAM is typically diffused throughout the cell, or associated with vacuoles and/or ingested material, and nearly uniform throughout the whole cell, but not all CAML express EpCAM, because some tumors express very low or no EpCAM.
- If CAMLs express a marker, the marker is typically diffused throughout the cell, or associated with vacuoles and/or ingested material, and nearly uniform throughout the whole cell, but not all CAML express the same markers with equal intensity.
- CAMLs often express markers associated with the markers of the tumor origin; e.g., if the tumor is of prostate cancer origin and expresses PSMA, then CAMLs from such a patient also expresses PSMA. As another example, if the primary tumor is of pancreatic origin and expresses PDX-1, then CAMLs from such a patient also expresses PDX-1. As further example, if the primary tumor or CTC of the cancer origin express CXCR-4, then CAMLs from such a patient also express CXCR-4.
- If the primary tumor or CTC originating from the cancer expresses a biomarker of a drug target, CAMLs from such a patient also express the biomarker of the drug target. An example of such a biomarker of immunotherapy is PD-L1.
- CAMLs express monocytic markers (e.g. CD11c, CD14) and endothelial markers (e.g. CD146, CD202b, CD31).
- CAMLs have the ability to bind Fc fragments.

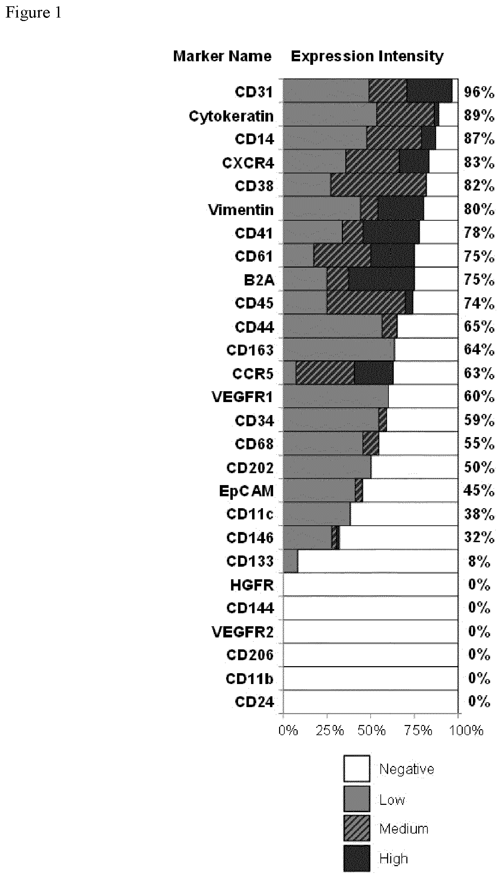

An extensive set of markers were evaluated for their expression on CAMLs, and the results are shown in FIG. 1. In one aspect of the invention, the CAMLs of the present invention express 1, 2, 3, 4, 5, 6, 7, 8, 9, 10, 11, 12, 13, 14, 15, 16, 17, 18, 19, 20 or all 21 of the markers shown in Figure 1. The markers were screened against 1118 CAMLs from 93 different patients with different cancers. CAMLs were initially isolated and identified with DAPI, cytokeratin, and CD45; then sequentially restained with total of 27 markers including myeloid/macrophage, white blood cell, megakaryocyte, epithelial, endothelial, progenitor/stem, and motility markers. As can be seen from Fig. 1, marker expression ranges from 0% to 96%. Almost all CAMLs were found to express levels of CD31, and commonly co-expressed cytokeratin, CD14, CXCR4, vimentin and other markers. However, while CAMLs contained clear myeloid lineage marker (CD14), CD31 marker was expressed more often at 96%.

CAMLs also present with numerous phenotypes which do not appear to match the understanding of classical cellular differentiation (i.e. co-expression of CD45 [leukocyte] and cytokeratin [epithelial], CD11c [macrophage] and CD41 [megakaryocyte], CD146 [endothelial] and CD41/CD61 [megakaryocyte], CD41/CD61 [megakaryocyte] and CD68/CD163 [scavenger macrophage]). Many of the markers appear on multiple cell types. Combined, these data show CAMLs are myeloid-derived cells early in their differentiation process that possess many phenotypic attributes associated with stem cell and proangiogenic capabilities.

CAMLs can be visualized by colorimetric stains, such as H&E, or fluorescent staining of specific markers as shown in FIG. 1. For the cytoplasm, CD31 is the most positive phenotype. CD31 alone, or in combination with other positive markers in FIG. 1, or cancer markers associated with the tumor are recommended.

In the various embodiments and aspects of the invention, the circulating cells and CAMLs can be defined as cells having the following characteristics: (a) a large atypical polyploid nucleus of about 14-64 µm in size, or multiple nuclei in a single cell; (b) cell size of about 20-300 microns in size; and (c) a morphological shape selected from the group consisting of spindle, tadpole, round, oblong, two legs, more than two legs, thin legs, and amorphous. In further embodiments, the CAMLs can be defined as also having one or more of the following additional characteristics: (d) CD14 positive phenotype; (e) CD45 expression; (f) EpCAM expression; (g) vimentin expression; (h) PD-L1 expression; (i) monocytic CD11C marker expression; (j) endothelial CD146 marker expression; (k) endothelial CD202b marker expression; and (l) endothelial CD31 marker expression.

As suggested above, the unique characteristics of the CAMLs and CTCs described herein make them well-suited for use in clinical methodology including methods of screening and diagnosis diseases such as cancer, monitoring treatment, monitoring of disease progression and recurrence.

### Predicting OS and PFS Using Cell Size

As suggested in the Summary above, the present invention is directed to methods for predicting overall survival (OS) and progression free survival (PFS) of a subject having cancer based on circulating cell size. These methods comprise determining the size of circulating cells in a biological sample from a first subject having cancer, and comparing the results with a second subject having cancer. The OS and PFS of the subject with larger-sized cells is predicted to be less than the OS and PFS of the subject having smaller-sized cells.

In preferred aspects of the invention, the methods comprise determining the size of circulating cells in a biological sample from a subject having cancer, wherein when at least one cell in said sample is about 50 µm or more in size, the OS and PFS of the subject is predicted to be less than a subject having cancer where none of the cells is more than about 50 µm in size.

The 50 µm value can be considered a cut-off value for this method. In related aspects of this method, the cut-off value may be any one of 40, 41, 42, 43, 44, 45, 46, 47, 48, 49, 50, 51, 52, 53, 54, 55, 56, 57, 58, 59, or 60 µm or more.

In one aspect of the invention, the methods of the invention comprise determining the size of circulating cells in a biological sample from a subject having cancer, wherein when at least one cell in said sample is about 50 µm or more in size, the OS and PFS of the subject is predicted to be less than a subject having cancer where none of the cells is more than about 50 µm in size.

With respect to this method, it is important to recognize that the size of a particular circulating cell can vary depending on the condition and morphology of the cell, the circumstances under which the size is determined, and the manner in which the size is determined. Because the types of morphologies adopted by the circulating cells include spindle, tadpole, round, oblong, two legs, etc. (as further defined herein), the size can also vary depending on the two points on a cell selected for measurement. However, the size of the cell will generally be measured between the two points most distant on the body of the cell. Thus, if the shape is round, the diameter of the cell is measured. If the shape is spindle, the distance between the two ends along the axial length of the cell can be measured.

In each aspect of this embodiment, the circulating cells can also be termed CAMLs.

### Predicting OS and PFS Using Cell Number

The present invention is also directed to methods for predicting OS and PFS of a subject having cancer based on circulating cell numbers. These methods comprise determining a ratio of circulating cells in a selected volume of a biological sample from a subject having cancer, wherein when said ratio is equivalent to about 4 or more cells per 7.5 mL of the biological sample, the OS and PFS of the subject is predicted to be less than a subject having cancer with a ratio equivalent to less than 4 cells per 7.5 mL of the biological sample.

The 4 circulating cells value can be considered a cut-off value for this method. In related aspects of this method, the cut-off value may be any one of 4, 4.5, 5, 5.5, 6, 6.5, 7, 7.5, 8, 8.5, 9, 9.5 or 10 cells or more per 7.5 mL of the biological sample.

As a further example, the methods of the invention comprise determining a ratio of circulating cells in a selected volume of a biological sample from a subject having cancer, wherein when said ratio is equivalent to about 6 or more cells per 7.5 mL of the biological sample, the OS and PFS of the subject is predicted to be less than a subject having cancer with a ratio equivalent to less than 6 cells per 7.5 mL of the biological sample.

In each aspect of this embodiment, the circulating cells can also be termed CAMLs.

### Predicting OS and PFS Using CEP17 Dot Number

The present invention is further directed to methods for predicting OS and PFS of a subject having cancer based on the number of CEP17 dots detected in circulating cells. These methods comprise determining the number of CEP17 dots in circulating cells in a biological sample from a subject having cancer, wherein when at least one cell in said sample has about 8 or more CEP17 dots, the OS and PFS of the subject is predicted to be less than a subject having cancer where none of the cells in a biological sample from the subject has about 8 or more CEP17 dots.

To avoid any confusion, the phrase "none of the cells in a biological sample from the subject has about 8 or CEP17 dots" should be understood to mean that each of the cells in the biological sample has 7 or fewer CEP17 dots.

The "8 or more" CEP17 dots value can be considered a cut-off value for this method. In related aspects of this method, the cut-off value may be any one of 8 or more, 9 or more, 10 or more, 11 or more, 12 or more, 13 or more, 14 or more, or 15 or more CEP17 dots or more.

In one aspect of the invention, the methods of the invention comprise determining the number of CEP17 dots in circulating cells in a biological sample from a subject having cancer, wherein when at least one cell in said sample has about 10 or more CEP17 dots, the OS and PFS of the subject is predicted to be less than a subject having cancer where none of the cells in a biological sample from the subject has about 10 or more CEP17 dots. To avoid any confusion, the phrase "none of the cells in a biological sample from the subject has about 10 or CEP17 dots" should be understood to mean that each of the cells in the biological sample has 9 or fewer CEP17 dots.

In each aspect of this embodiment, the circulating cells can also be termed CAMLs.

In each of the methods of the invention, overall survival (OS) or progression free survival (PFS), or both, is over a period of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 months, or more. In one aspect of the invention, OS or PFS, or both, is over a period of at least about 24 months.

As used herein, overall survival (OS) means the length of time survived by a subject having cancer from a selected date, such as the date of diagnosis, the date on which treatment began, and the date on which blood is drawn to assess cancer progression.

As used herein, progression free survival (PFS) means the length of time survived by a subject having cancer from a selected date, such as the date on which treatment began or the date on which blood is drawn to assess cancer progression, and where the cancer has not worsened or progressed.

In each of the methods of the invention, it will be apparent that the amount of the biological sample in which the circulating cells (CAMLs) are assayed can vary. However, to obtain a relevant number of cells when the methods are based on determining the size of the cells, the biological sample should be at least about 2.5 mL. The amount of biological sample may also be at least about 3, 4, 5, 6, 7, 7.5, 8, 9, 10, 11, 12, 12.5, 13, 14, 15, 16, 17, 17.5, 18, 19, 20, 21, 22, 22.5, 23, 24, 25, 26, 27, 27.5, 28, 29, or 30 mL, or more. The amount of biological sample may also be between about 2.5 and 20 mL, between about 5 and 15 mL, or between about 5 and 10 mL. In one aspect of the invention, the biological sample is about 7.5 mL.

With respect to methods for predicting OS and PFS of a subject having cancer based on circulating cell (CAML) numbers in a biological sample, a ratio of circulating cells (CAMLs) in a selected volume of a biological sample from a subject having cancer is determined. It will be understood that various amounts of the biological sample can be used, but that a ratio of the number of cells in the sample to the size of the sample is determined and compared between subjects. For example, a ratio 8 cells in 15 mL of a sample is equivalent to 4 cells in 7.5 mL of a sample, and to 2 cells in 3.75 mL of a sample.

In each of the embodiments and aspects of the invention, the circulating cells (CAMLs) can be defined as having each of the following characteristics:
(a) large atypical polyploid nucleus of about 14-64 µm in size, or multiple nuclei in the same cell;
(b) cell size of about 20-300 µm in size; and
(c) morphological shape selected from the group consisting of spindle, tadpole, round, oblong, two legs, more than two legs, thin legs, and amorphous.

In certain aspects of the embodiments of the invention, the circulating cells (CAMLs) can be further defined as having one or more of the following additional characteristics:
(d) CD14 positive phenotype;
(e) CD45 expression;
(f) EpCAM expression;
(g) vimentin expression;
(h) PD-L1 expression;
(i) monocytic CD11C marker expression;
(j) endothelial CD146 marker expression;
(k) endothelial CD202b marker expression; and
(l) endothelial CD31 marker expression.

In each of the embodiments and aspects of the invention, the source of the biological sample may be, but is not limited to, one or more of peripheral blood, blood, lymph node, bone marrow, cerebral spinal fluid, tissue, and urine. When the biological sample is blood, the blood may be antecubital-vein blood, inferior-vena-cava blood femoral vein blood, portal vein blood, or jugular-vein blood, for example. The sample may be a fresh sample or a cryo-preserved sample that is thawed [8].

When comparing the circulating cells (CAML) sizes between two subjects having cancer, the subjects to have the same type of cancer. However, it may be difficult to fully match two subjects in terms of the stage of the cancer, the rate of progression of the cancer, history of treatment, and history of remission and/or reoccurrence of the cancer, among other factors. Therefore, it should be understood that there may be some variations in the characteristics in the cancers of two subject that are being compared in this method.

In each of the embodiments and aspects of the invention, the cancer is a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer. The skilled artisan will appreciate that the methods of the invention are not limited to particular forms or types of cancer and that they may be practiced in association with a wide variety of cancers.

In each of the embodiments and aspects of the invention, the circulating cells (CAMLs) are isolated from the biological samples for the determining steps using one or more means selected from size exclusion methodology, immunocapture, red blood cell lysis, white blood cell depletion, FICOLL density gradient, electrophoresis, dielectrophoresis, flow cytometry, magnetic levitation, and various microfluidic chips, or a combination thereof. In a particular aspect, the size exclusion methodology comprises use of a microfilter.

In one aspect of the invention, circulating cells (CAMLs) are isolated from the biological samples using size exclusion methodology that comprises using a microfilter. Suitable microfilters can have a variety of pore sizes and shapes. The microfilter may have a pore size ranging from about 5 microns to about 20 microns. In certain aspects of the invention, the pore size is between about 5 and 10 microns; in other aspects, the pore size is between about 7 and 8 microns. The larger pore sizes will eliminate most of the WBC contamination on the filter. The pores of the microfilter may have a round, race-track shape, oval, square and/or rectangular pore shape. The microfilter may have precision pore geometry and/or uniform pore distribution.

In another aspect of the invention, circulating cells (CAMLs) are isolated from the biological samples using a microfluidic chip via physical size-based sorting, hydrodynamic size-based sorting, grouping, trapping, immunocapture, concentrating large cells, or eliminating small cells based on size. The circulating cell (CAML) capture efficiency can vary depending on the collection method. The circulating cell (CAML) size that can be captured on different platforms can also vary. The principle of using circulating cell size to determine prognosis and survival is the same, but the statistics will vary. Collection of circulating cells using CellSieve^{™} microfilters provides 100% capture efficiency and high quality cells.

In another aspect of the invention is the blood collection tube. CellSave blood collection tubes (Menarini Silicon Biosystems Inc., San Diego, CA) provide stable cell morphology and size. Other available blood collection tubes do not provide cell stability. Cells can enlarge and may even burst collect in most other blood collection tubes.

In another aspect of the invention is to identify large cells in the sample without specifically identifying the cells as CAML cells *per se*, instead simply identifying the cells based on size of the cytoplasm and nucleus. Examples are techniques using color metric stains, such as H&E stains, or just looking at CK (+) cells.

In a further aspect of the invention, circulating cells (CAMLs) are isolated from the biological samples using a CellSieve^{™} low-pressure microfiltration assay.

### Examples

### Sample collection and processing

In each of the Examples provided herein and outlined below, peripheral blood was collected in CellSave Tubes (Menarini Silicon Biosystems Inc., San Diego, CA) and processed within 96 hours. CellSieve^{™} microfiltration technique was used to collect all cancer associated cells (CTCs, EMTs, CECs, and CAMLs) in the blood sample. CellSieve^{™} microfilters have greater than 160,000 pores in uniform array with 7 µm pore diameter within a 9 mm area. The reagents include prefixation buffer, postfixation buffer, permeabilization buffer, and an antibody cocktail. The technique to perform the filtration used either a syringe pump set drawn at 5 mL/min [6] or a vacuum pump [4]. The filtration process started by prefixing 7.5 mL of the blood in 7.5 mL of prefixation buffer before drawn through the filter. The filter and captured cells were then subjected to washing, postfixation, washing, permeabilization and washing. Then, the captured cells on the filter were stained with an antibody cocktail consisting of FITC-anti-Cytokeratin 8, 18, 19; Phycoerythrin (PE) conjugated EpCAM; and Cy5-anti-CD45(5), followed by wash. Filters were placed onto a microscope slide and cover-slipped with Fluoromount-G/DAPI (Southern Biotech). An Olympus BX54WI Fluorescent microscope with Carl Zeiss AxioCam was used to image cells using specific fluorescent cubes and monochrome camera. Exposures were preset as 3 sec (Cy5), 2 sec (PE), 100-750 msec (FITC), and 10-50 msec (DAPI) for equal signal comparisons between cells. A Zen2011 Blue (Carl Zeiss) was used to process the images.

Because some cancers do not always exhibit high expressions of CK and EpCAM, cells on the filter were restained with CD31 and/or CD14 to improve CAML counts. The restaining technique was as published [5]. The cells were than reimaged. CD31 increased the CAML count for lung cancer.

### Frequency of CTCs and CAMLs in Blood of Cancer Patients

PDCTCs are rarely found in early stages of cancer. Even though PDCTCs are found more frequently in stage III and IV breast and prostate cancer patients, they are seen at low frequencies in most other solid tumors. CAMLs are found more commonly.

As an example, 7.5 mL of peripheral whole blood was obtained from 293 cancer patients (breast (n=59), prostate (n=52), pancreatic (n=59), lung (n=59), esophageal (n=27), and renal cell carcinoma (n=37)) and 30 health controls. The samples were analyzed using CellSieve^{™} microfiltration assay.

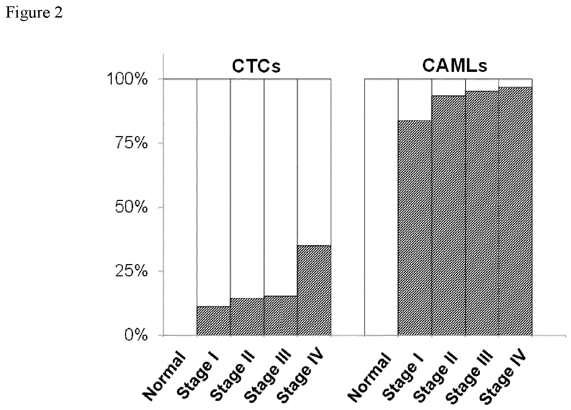

As can be seen from **FIG. 2****,** CAMLs were found to be more common than PDCTCs. CAMLs were found in 93% of patient samples, including pre-metastatic patients in Stage I (n=60) (84%), Stage II (n=62) (94%), Stage III (n=65) (95%), and in metastatic Stage IV (n=97) (97%) patients and 9 unstaged (undetermined), but absent in all healthy controls. Thus, CAMLs are found in high percentages in early stages of solid tumors, while CTCs were not.

### CAML Number and Size Variation as a Function of Stage

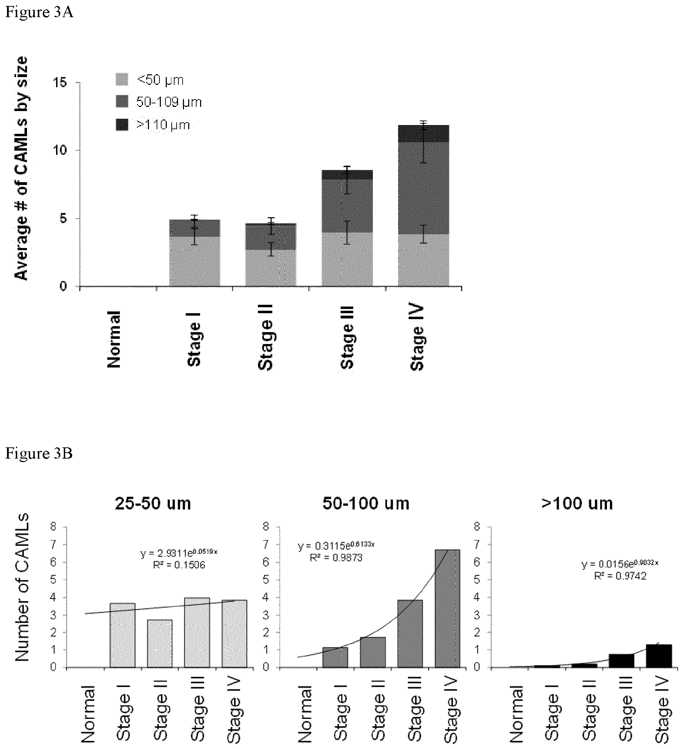

**FIGS. 3A** and **3B** analyzed CAML number and size based on stage for samples from **FIG. 2****.** The average number of CAMLs increases as the stage increase. The average number of CAMLs with the size, end to end, between 25-50 µm is roughly the same for all stages. However, the average number of CAML sizes 50-100 µm and size >100 µm increases with stage as shown in **FIG. 3B****.**

### Correlation of CAML Size with Number of CEP17 Dots in the Nuclear Area

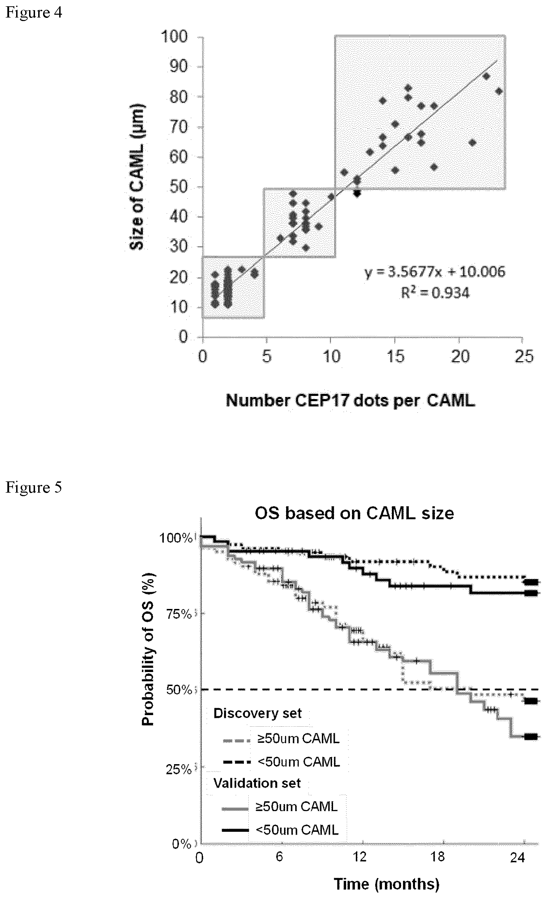

Increased CAML size was postulated to be a result of CAML engulfing of tumor cells and tumor material. Indeed, one experiment demonstrated that the number of chromosome enumeration probe 17 (CEP17) dots, an indicator of number of nuclei engulfed, correlated well with the size of the cells. Fig. 4 shows that CAMLs >50 µm in size have more CEP17 dots than CAMLs of between 25-50 µm in size. The data also suggests that a CAML size of 50 µm is a dividing line consistent with the results on disease progression presented in Fig. 3B.

### Development of Size Criteria to Determine Aggressiveness of Disease

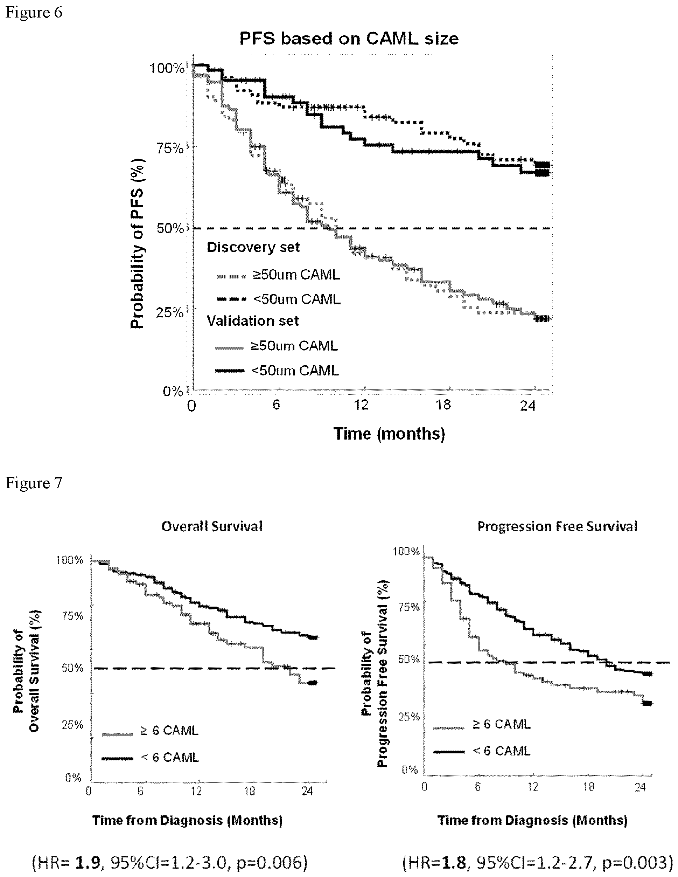

**FIGS. 3** and **4** suggests that 50 µm may be use to separate aggressive disease. 7.5 mL of peripheral whole blood were analyzed using CellSieve^{™} microfiltration assay. A discovery set of 157 patients tracked over 24 months from a variety of cancers and stages were analyzed for OS, doted curves in **FIG. 5****,** and progressive free survival, doted curves in **FIG. 6****.** The hazard ratio for OS is 3.4 and 95%CI=2.1-5.6 with p<0.001. The hazard ratio for PFS is 3.2 and 95%CI=2.1-5.0 with p<0.001. This indicates that patients with CAML sizes ≥50 µm have shorter PFS and OS.

A validation set of 158 patients from a similar variety of cancers and stages were analyzed for OS, solid curves in **FIG. 5****,** and PFS, solid curves in **FIG. 6****.** The hazard ratio for OS is 4.2 and 95%CI=2.3-7.7 with p<0.001. The hazard ratio for PFS is 3.8 and 95%CI=2.7-6.8 with p<0.001. The validation results are in good agreement with the discovery results.

### Multivariate Analysis and Prognostic Information of CAML Size

Based on data shown in FIGS. 5 and 6, a multivariate analysis for OS and PFS was performed on n=293 patient samples described above. Patients were tracked over 24 months. A multivariate analysis was performed shown in the table below. It shows that the CAML size of 50 µm is the most important parameter to determine OS and PFS. The smaller the p value, the higher the probability the statistics is accurate.

**Table 1**

| **Variable** | **OS (p value)** | **PFS (p value)** |
|---|---|---|
| Stage | 0.866 | 0.751 |
| # CTCs (<5 vs ≥5) | 0.995 | 0.136 |
| # CAMLs (<6 vs ≥6) | 0.816 | 0.244 |
| **CAML Size (<50 µm vs ≥50 µm)** | **0.015** | **0.000** |
| Therapy chemo/targeted/none | 0.056 | 0.645 |
| Metastatic positive vs negative | 0.678 | 0.165 |
| Node negative, local, distant | **0.017** | 0.231 |
| Tumor Size (T1, T2, T3 or T4) | **0.012** | 0.485 |
| M/F | **0.036** | 0.796 |
| Age | 0.088 | 0.066 |
| Race Caucasian/Black/Asian/Hispanic) | 0.152 | 0.055 |

OS and PFS analysis based on CAML number are shown in **FIG. 7****.** This data shows that when the number of CAMLs in a 7.5 mL peripheral blood sample is ≥ 6 CAMLs, both OS and PFS are decreased in comparison to a patient having < 6 CAMLs in a sample of the same size.

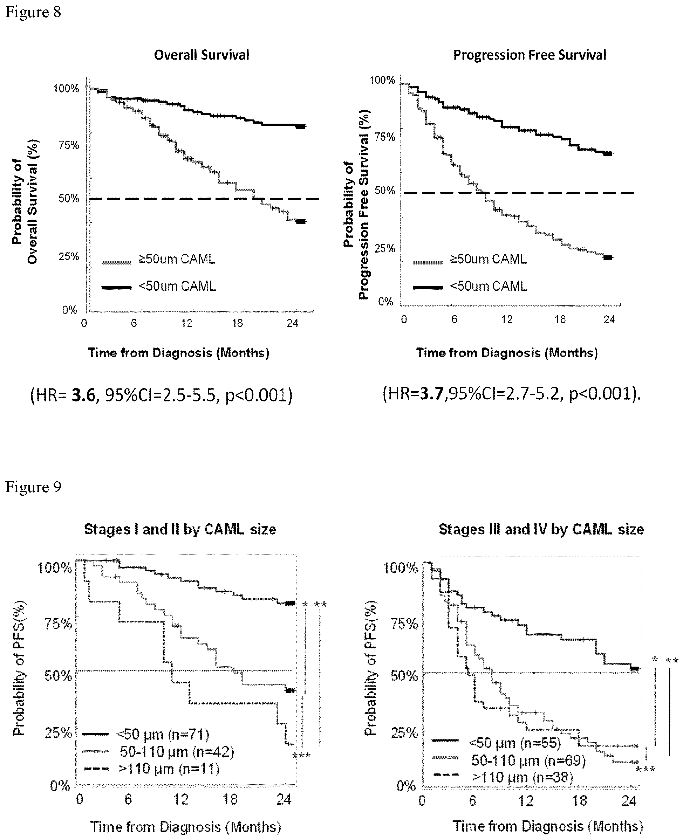

Further data associated with OS and PFS are presented in **FIG. 8****.** The data presented in **FIG. 8** is based on CAML size: ≥ 50 µm versus < 50 µm. As indicated by the p value, CAML size ≥ 50 µm is very important in predicting poor prognosis.

**FIG. 9** separated the plots into three different CAML sizes: < 50 µm, 50-100 µm and > 100 µm. It is clear that larger the CAML size, the worse the prognosis. It is also important to note that the CAML size is important in determining prognosis even for Stages I and II (left figure).

A larger sample size of n=315 was used to allow demonstration the utility of CAML size to predict survival and progression concept in all stages and the various cancers. The 315 cancer patients are from (breast (n=59), prostate (n=74), pancreatic (n=59), lung (n=59), esophageal (n=27), and renal cell carcinoma (n=37)). They are in Stage I (62), Stage II (72), Stage III (n=69), Stage IV (106), and 6 unstaged.

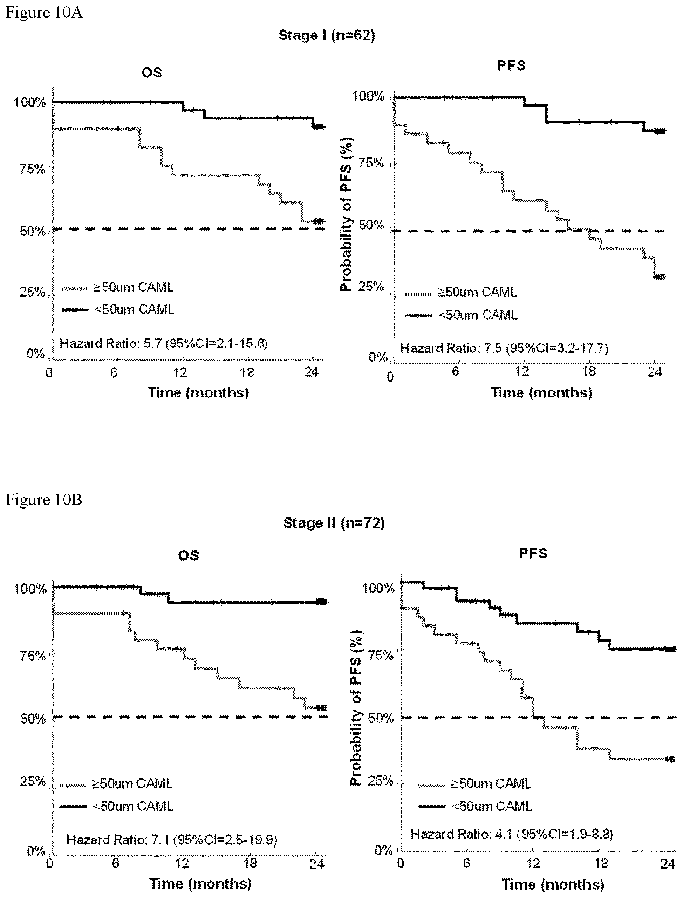

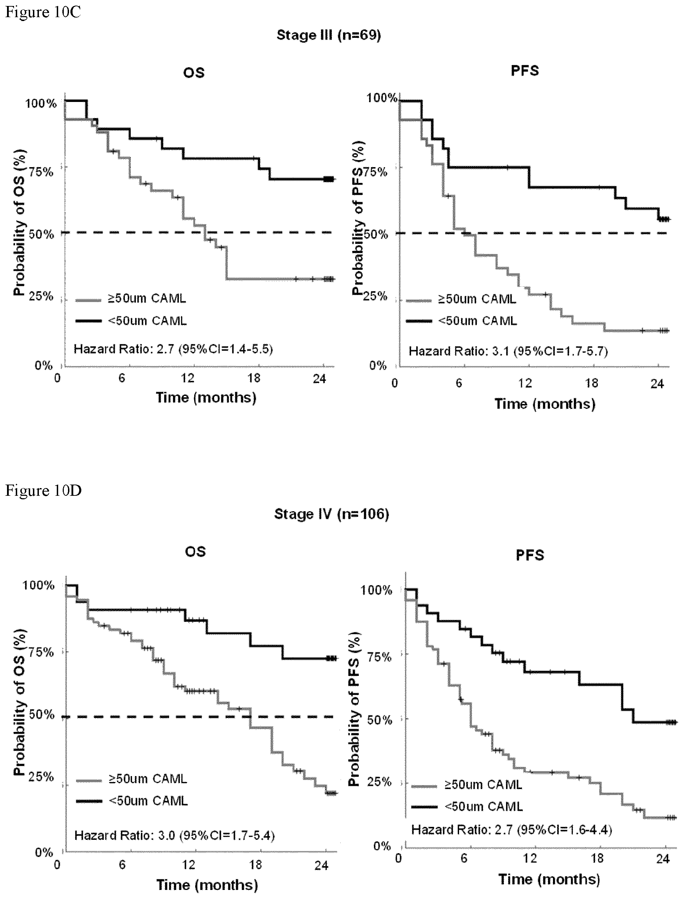

**FIGS. 10A-10D** provide detailed information of OS (left figure) and PFS (right figure) for CAML sizes ≥ 50 µm and < 50 µm for Stages I, II, III and IV, respectively.

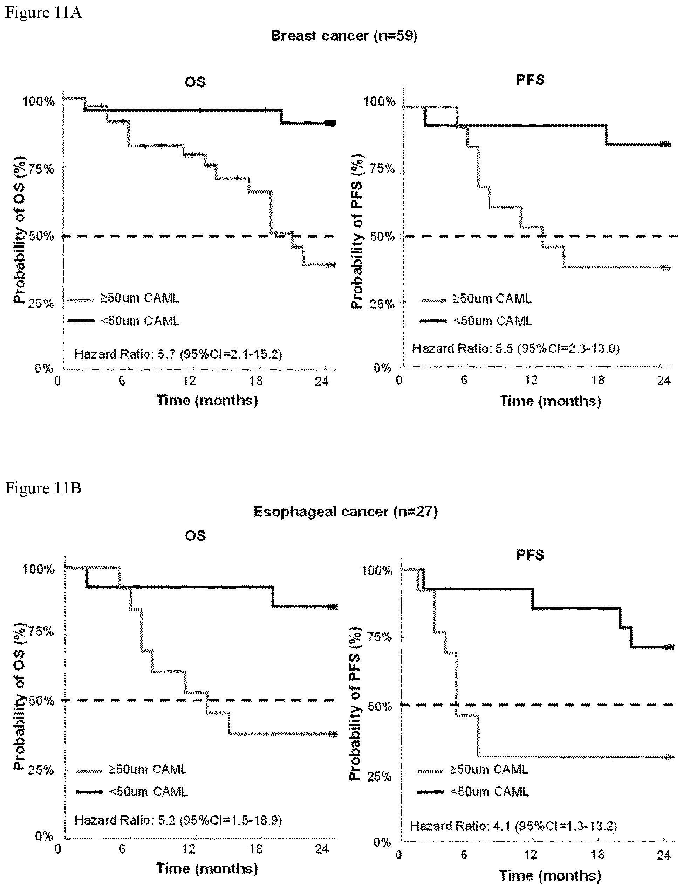

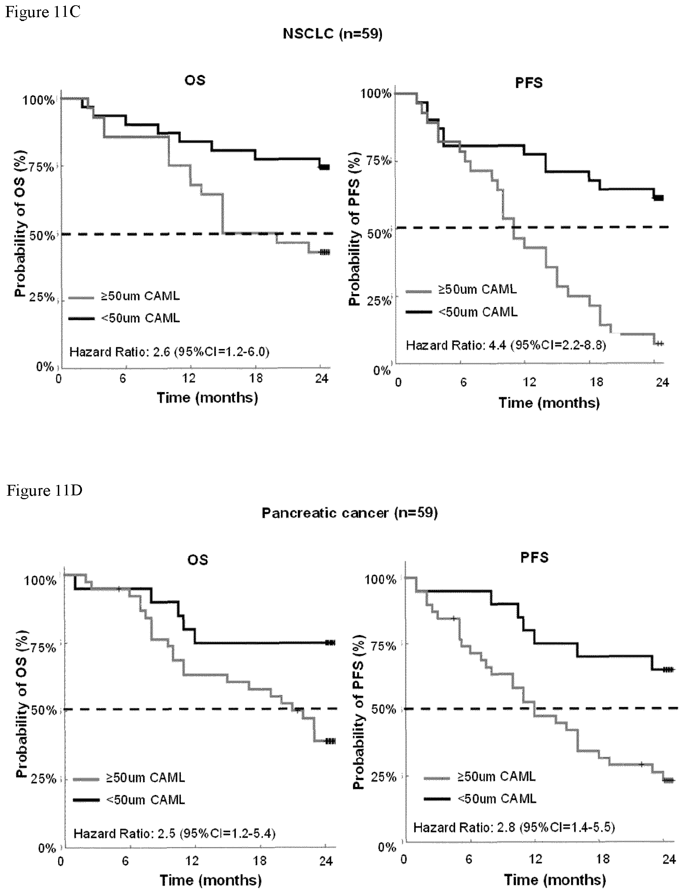

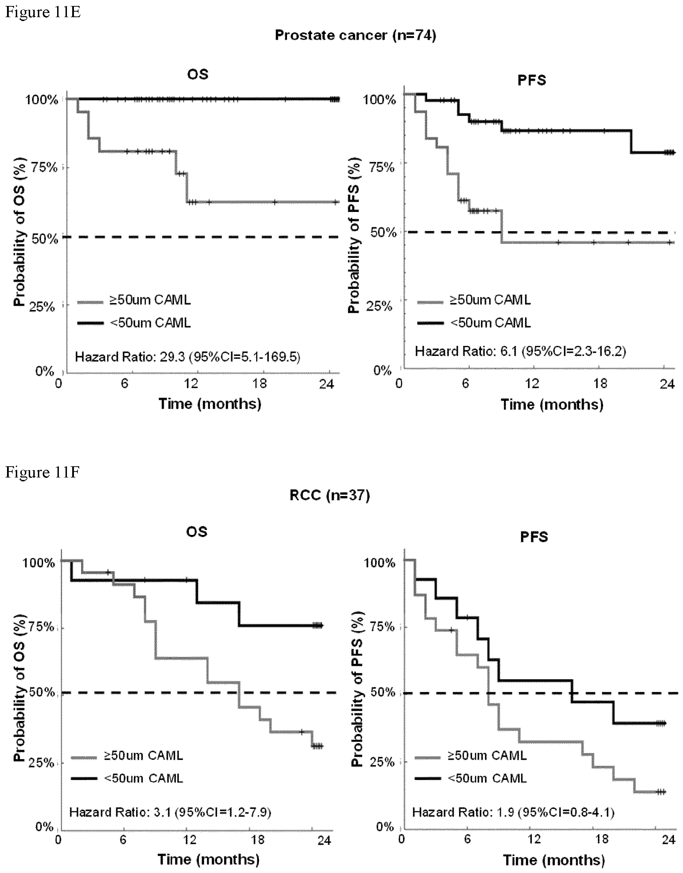

**FIGS. 11A-11F** provide detailed information of OS (left figure) and PFS (right figure) for CAML sizes ≥ 50 µm and < 50 µm for breast cancer, esophageal cancer, non-small cell lung cancer (NSCLC), pancreatic cancer, prostate cancer, and renal cell carcinoma, respectively. By stage or by cancer, CAML size > 50 µm indicates poor OS and PFS. The optimal size cutoff can change slightly depending on the type of cancer for optimal p value.

### CITATIONS

1. Adams, D., et al., Circulating giant macrophages as a potential biomarker of solid tumors. PNAS 2014, 111(9):3514-3519.
2. International Patent Application Publication No. WO 2013/181532, dated December 5, 2013.
3. Adams, D. L., et al., Cytometric characterization of Circulating Tumor Cells captured by microfiltration and their correlation to the CellSearch® CTC test. Cytometry Part A 2015; 87A:137-144.
4. Adams DL, et al. The systematic study of circulating tumor cell isolation using lithographic microfilters. RSC Adv 2014, 4:4334-4342.
5. Adams et al., Multi-phenotypic subtyping of circulating tumor cells using sequential fluorescent quenching and restaining, Scientific Reports 2016, 6:33488 1 DOI: 10.103 8/srep33488.
6. International Patent Application Publication No. WO 2013/078409, dated May 30, 2013.
7. International Patent Application Publication No. WO 2016/33103, dated March 3, 2016.
8. Zhu P, et al., Detection of Tumor-Associated Cells in Cryopreserved Peripheral Blood Mononuclear Cell Samples for Retrospective Analysis, J of Translational Medicine, 2016, 14:198. DOI: 10.1186/s12967-016-0953-2.

## Claims

1. A method for predicting overall survival (OS) and progression free survival (PFS) of a subject having cancer, said method comprising determining the size of circulating cells (CAMLs) in a biological sample from a first subject having cancer, and comparing the results with a second subject having cancer, wherein the OS and PFS of the subject with larger-sized cells is predicted to be less than the OS and PFS of the subject having smaller-sized cells, wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

2. A method for predicting OS and PFS of a subject having cancer, said method comprising determining the size of circulating cells (CAMLs) in a biological sample from a subject having cancer, wherein when at least one cell in said sample is about 50 µm or more in size, the OS and PFS of the subject is predicted to be less than a subject having cancer where none of the cells is more than about 50 µm in size, wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

3. A method for predicting OS and PFS of a subject having cancer, said method comprising determining a ratio of circulating cells (CAMLs) in a selected volume of a biological sample from a subject having cancer, wherein when said ratio is equivalent to about 6 or more cells per 7.5 mL of the biological sample, the OS and PFS of the subject is predicted to be less than a subject having cancer with a ratio equivalent to less than 6 cells per 7.5 mL of the biological sample wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

4. A method for predicting OS and PFS of a subject having cancer, said method comprising determining the number of CEP17 dots in circulating cells (CAMLs) in a biological sample from a subject having cancer, wherein when at least one cell in said sample has about ten or more CEP17 dots, the OS and PFS of the subject is predicted to be less than a subject having cancer where none of the cells in a biological sample from the subject has about ten or more CEP17 dots, wherein the circulating cells have the following characteristics:
(a) large atypical polyploid nucleus of about 14-64 µm in size, or multiple nuclei in a single cell;
(b) cell size of about 20-300 µm in size; and
(c) morphological shape selected from the group consisting of spindle, tadpole, round, oblong, two legs, more than two legs, thin legs, and amorphous.

5. The method of any one of claims 1-4, wherein said OS and/or PFS is over a period of at least 12 months, preferably over a period of at least 24 month.

6. The method of any one of claims 1-4, wherein the size of the biological sample is between 5 and 15 mL.

7. The method of any one of claims 1-4, wherein the circulating cells have one or more of the following additional characteristics:
(d) CD14 positive phenotype;
(e) CD45 expression;
(f) EpCAM expression;
(g) vimentin expression;
(h) PD-L1 expression;
(i) monocytic CD11C marker expression;
(j) endothelial CD146 marker expression;
(k) endothelial CD202b marker expression; and
(l) endothelial CD31 marker expression.

8. The method of any one of claims 1-4, wherein the source of the biological sample is one or more of peripheral blood, blood, lymph node, bone marrow, cerebral spinal fluid, tissue, and urine.

9. The method of claim 8 wherein the biological sample is antecubital-vein blood, inferior-vena-cava blood, femoral vein blood, portal vein blood, or jugular-vein blood.

10. The method of any one of claims 1-4, wherein the cancer is a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer.

11. The method of any one of claims 1-4, wherein circulating cells are isolated from the biological samples for the determining steps using one or more means selected from the group consisting of size exclusion methodology, immunocapture, red blood cell lysis, white blood cell depletion, FICOLL density gradient, electrophoresis, dielectrophoresis, flow cytometry, magnetic levitation, and various microfluidic chips, or a combination thereof.

12. The method of claim 11, wherein circulating cells are isolated from the biological samples using size exclusion methodology that comprises using a microfilter.

13. The method of claim 12, wherein the microfilter has a pore size ranging from about 5 microns to about 20 microns, wherein the pores of the microfilter have a round, race-track shape, oval, square and rectangular pore shape, wherein the microfilter has precision pore geometry and uniform pore distribution.

14. The method of claim 11, wherein circulating cells are isolated using a microfluidic chip via physical size-based sorting, hydrodynamic size-based sorting, grouping, trapping, immunocapture, concentrating large cells, or eliminating small cells based on size.

15. The method of any one of claims 1-4, wherein circulating cells are isolated from the biological samples for the determining steps using a low-pressure microfiltration assay with microfilters have greater than 160,000 pores in uniform array with 7 µ m pore diameter within a 9 mm area.

## Patentansprüche

1. Ein Verfahren zur Vorhersage der Gesamtüberlebenszeit (engl. overall survival, OS) und der progressionsfreien Überlebenszeit (engl. progression free survival, PFS) eines Probanden mit Krebserkrankung,
wobei besagtes Verfahren das Bestimmen der Größe zirkulierender Zellen (CAMLs) in einer biologischen Probe von einem ersten Probanden mit Krebserkrankung und das Vergleichen der Ergebnisse mit einem zweiten Probanden mit Krebserkrankung umfasst,
wobei die OS und PFS von dem Probanden mit den größeren Zellen als kleiner vorausgesagt ist als die OS und PFS von dem Probanden mit den kleineren Zellen, wobei die zirkulierenden Zellen folgende Charakteristika haben:
(a) großer atypischer polyploider Zellkern mit einer Größe von etwa 14 - 64 µm, oder mehrere Zellkerne in einer einzigen Zelle;
(b) Zellgröße mit einer Größe von etwa 20 - 300 µm; und
(c) morphologische Form ausgewählt aus einer Gruppe umfassend Spindel, Kaulquappe, rund, länglich, mit zwei Beinen, mit mehr als zwei Beinen, mit dünnen Beinen und amorph.

2. Ein Verfahren zur Vorhersage der OS und der PFS eines Probanden mit Krebserkrankung, wobei besagtes Verfahren das Bestimmen der Größe zirkulierender Zellen (CAMLs) in einer biologischen Probe von einem Probanden mit Krebserkrankung umfasst,
wobei wenigstens eine Zelle in besagter Probe mindestens eine Größe von etwa 50 µm hat,
die OS und die PES des Probanden als niedriger vorausgesagt ist als bei einem Probenaden, bei dem keine der Zellen größer als etwa 50 µm ist,
wobei die zirkulierenden Zellen folgende Charakteristika haben:
(a) großer atypischer polyploider Zellkern mit einer Größe von etwa 14 - 64 µm, oder mehrere Zellkerne in einer einzigen Zelle;
(b) Zellgröße mit einer Größe von etwa 20 - 300 µm; und
(c) morphologische Form ausgewählt aus einer Gruppe umfassend Spindel, Kaulquappe, rund, länglich, mit zwei Beinen, mit mehr als zwei Beinen, mit dünnen Beinen und amorph.

3. Ein Verfahren zur Vorhersage der OS und der PFS eines Probanden mit Krebserkrankung, wobei besagtes Verfahren das Bestimmen eines Verhältnisses von zirkulierenden Zellen (CAMLs) in einem ausgewählten Volumen einer biologischen Probe eines Probanden mit Krebserkrankung umfasst,
wobei die OS und die PFS des Probenaden, bei dem das Verhältnis etwa 6 oder mehr Zellen pro 7.5 mL der biologischen Probe entspricht, als niedriger vorausgesagt ist als bei einem Probanden mit Krebserkrankung bei dem das Verhältnis kleiner 6 Zellen pro 7.5 mL der biologischen Probe entspricht,
wobei die zirkulierenden Zellen folgende Charakteristika haben:
(a) großer atypischer polyploider Zellkern mit einer Größe von etwa 14 - 64 µm, oder mehrere Zellkerne in einer einzigen Zelle;
(b) Zellgröße mit einer Größe von etwa 20 - 300 µm; und
(c) morphologische Form ausgewählt aus einer Gruppe umfassend Spindel, Kaulquappe, rund, länglich, mit zwei Beinen, mit mehr als zwei Beinen, mit dünnen Beinen und amorph.

4. Ein Verfahren zur Vorhersage der OS und der PFS eines Probanden mit Krebserkrankung,
wobei besagtes Verfahren das Bestimmen der Anzahl von CEP17-Punkten in zirkulierenden Zellen (CAMLs) in einer biologischen Probe von einem Probanden mit Krebserkrankung umfasst,
wobei die OS und die PFS des Probanden, bei dem mindestens eine Zelle in besagter Probe etwa zehn oder mehr CEP17-Punkte aufweist, als niedriger vorausgesagt ist als bei einem Probanden mit Krebserkrankung bei dem keine der Zellen einer biologischen Probe des Probanden etwa zehn oder mehr CEP17-Punkte aufweist,
wobei die zirkulierenden Zellen folgende Charakteristika haben:
(a) großer atypischer polyploider Zellkern mit einer Größe von etwa 14 - 64 µm, oder mehrere Zellkerne in einer einzigen Zelle;
(b) Zellgröße mit einer Größe von etwa 20 - 300 µm; und
(c) morphologische Form ausgewählt aus einer Gruppe umfassend Spindel, Kaulquappe, rund, länglich, mit zwei Beinen, mit mehr als zwei Beinen, mit dünnen Beinen und amorph.

5. Das Verfahren nach einem der Ansprüche 1 bis 4, wobei besagte OS und/oder PFS über einen Zeitraum von mindestens 12 Monaten, vorzugsweise über einen Zeitraum von mindestens 24 Monaten ist.

6. Das Verfahren nach einem der Ansprüche 1 bis 4, wobei die Größe der biologischen Probe zwischen 5 und 15 mL ist.

7. Das Verfahren nach einem der Ansprüche 1 bis 4, wobei die zirkulierenden Zellen eine oder mehrere der folgenden zusätzlichen Charakteristika haben:
(d) CD14-positiver Phänotyp;
(e) CD45 Expression;
(f) EpCAM Expression;
(g) Vimentin Expression;
(h) PD-L1 Expression;
(i) monozytäre CD11C-Marker Expression;
(j) endotheliale CD146-Marker Expression;
(k) endotheliale CD202b-Marker Expression; und
(l) endotheliale CD31-Marker Expression.

8. Das Verfahren nach einem der Ansprüche 1 bis 4, wobei die Quelle der biologischen Probe ein oder mehrere von peripheren Blut, Blut, Lymphknoten, Knochenmark, zerebrale Rückenmarksflüssigkeit, Gewebe und Urin ist.

9. Das Verfahren nach Anspruch 8, wobei die biologische Probe Blut der Ellenbeugenvene, Blut der unteren Hohlvene, Blut der Oberschenkelvene, Blut der Pfortader, oder Blut aus der Halsvene ist.

10. Das Verfahren nach einem der Ansprüche 1 bis 4, wobei der Krebs ein fester Tumor, Krebs im Stadium I, Krebs im Stadium II, Krebs im Stadium III, Krebs im Stadium IV, Karzinom, Sarkom, Neuroblastom, Melanom, Epithelzellkrebs, Brustkrebs, Prostatakrebs, Lungenkrebs, Bauchspeicheldrüsenkrebs, Darmkrebs, Leberkrebs, Kopf- und Halskrebs, Nierenkrebs, Eierstockkrebs, Speiseröhrenkrebs oder ein anderer fester Tumorkrebs ist.

11. Das Verfahren nach einem der Ansprüche 1 bis 4, wobei zirkulierende Zellen aus den biologischen Proben für die Bestimmungsschritte unter Verwendung eines oder mehrerer Mittel isoliert werden, die aus der Gruppe ausgewählt sind, bestehend aus Größenausschlußverfahren, Immunocapture, Lyse roter Blutkörperchen, Abreicherung weißer Blutkörperchen, FICOLL-Dichtegradienten, Elektrophorese, Dielektrophorese, Durchflußzytometrie, magnetischer Levitation und verschiedenen mikrofluidischen Chips oder einer Kombination davon.

12. Das Verfahren nach Anspruch 11, wobei zirkulierende Zellen aus den biologischen Proben unter Verwendung einer Größenausschlussmethode isoliert werden, die die Verwendung eines Mikrofilters umfasst.

13. Das Verfahren nach Anspruch 12, wobei der Mikrofilter eine Porengröße im Bereich von etwa 5 Mikrometern bis etwa 20 Mikrometern aufweist, wobei die Poren des Mikrofilters eine runde, rennbahnförmige, ovale, quadratische und rechteckige Porenform aufweisen, wobei der Mikrofilter eine präzise Porengeometrie und eine gleichmäßige Porenverteilung aufweist.

14. Das Verfahren nach Anspruch 11, wobei zirkulierende Zellen durch die Verwendung von mikrofluidischen Chips durch physikalisch größenbasierte Sortierung, hydrodynamisch größenbasierte Sortierung, Gruppieren, Einfangen, Immunocapture, Konzentrieren großer Zellen, oder Eliminierung kleiner Zellen auf Basis der Größe isoliert werden.

15. Das Verfahren nach einem der Ansprüche 1 bis 4, wobei zirkulierende Zellen für die Bestimmungsschritte, unter Verwendung eines Niederdruck-Mikrofiltrationstests mit Mikrofiltern, die mehr als 160.000 Poren in gleichmäßiger Anordnung mit einem Porendurchmesser von 7 µm innerhalb einer Fläche von 9 mm aufweisen, aus den biologischen Proben isoliert werden.

## Revendications

1. Procédé de prédiction de la survie globale (SG) et de la survie sans progression (SSP) d'un sujet atteint d'un cancer, ledit procédé comprenant la détermination de la taille de cellules circulantes (CAML) dans un échantillon biologique d'un premier sujet atteint d'un cancer, et la comparaison des résultats avec un second sujet atteint d'un cancer, dans lequel la SG et la SSP du sujet présentant des cellules de plus grande taille est prédite pour être inférieure à la SG et à la SSP du sujet présentant des cellules de plus petite taille, dans lequel les cellules circulantes possèdent les caractéristiques suivantes :
(a) un grand noyau polyploïde atypique d'une taille d'environ 14-64 µm, ou plusieurs noyaux dans une seule cellule ;
(b) une taille cellulaire d'environ 20-300 µm ; et
(c) une forme morphologique sélectionnée dans le groupe consistant en une forme en fuseau, de têtard, ronde, oblongue, avec deux pieds, avec plus de deux pieds, avec des pieds fins, et amorphe.

2. Procédé de prédiction de la SG et la SSP d'un sujet atteint d'un cancer, ledit procédé comprenant la détermination de la taille de cellules circulantes (CAML) dans un échantillon biologique d'un sujet atteint d'un cancer, dans lequel lorsque au moins une cellule dans ledit échantillon possède une taille d'environ 50 µm ou plus, la SG et la SSP du sujet est prédite pour être inférieure à celle d'un sujet atteint d'un cancer chez qui aucune des cellules ne possède une taille supérieure à environ 50 µm, dans lequel les cellules circulantes possèdent les caractéristiques suivantes :
(a) un grand noyau polyploïde atypique d'une taille d'environ 14-64 µm, ou plusieurs noyaux dans une seule cellule ;
(b) une taille cellulaire d'environ 20-300 µm ; et
(c) une forme morphologique sélectionnée dans le groupe consistant en une forme en fuseau, de têtard, ronde, oblongue, avec deux pieds, avec plus de deux pieds, avec des pieds fins, et amorphe.

3. Procédé de prédiction de la SG et la SSP d'un sujet atteint d'un cancer, ledit procédé comprenant la détermination d'un ratio de cellules circulantes (CAML) dans un volume sélectionné d'un échantillon biologique d'un sujet atteint d'un cancer, dans lequel lorsque ledit ratio est équivalent à environ 6 cellules ou plus pour 7,5 ml de l'échantillon biologique, la SG et la SSP du sujet est prédite pour être inférieure à celle d'un sujet atteint d'un cancer ayant un ratio équivalent à moins de 6 cellules pour 7,5 ml de l'échantillon biologique, dans lequel les cellules circulantes possèdent les caractéristiques suivantes :
(a) un grand noyau polyploïde atypique d'une taille d'environ 14-64 µm, ou plusieurs noyaux dans une seule cellule ;
(b) une taille cellulaire d'environ 20-300 µm ; et
(c) une forme morphologique sélectionnée dans le groupe consistant en une forme en fuseau, de têtard, ronde, oblongue, avec deux pieds, avec plus de deux pieds, avec des pieds fins, et amorphe.

4. Procédé de prédiction de la SG et la SSP d'un sujet atteint d'un cancer, ledit procédé comprenant la détermination du nombre de points CEP17 dans des cellules circulantes (CAML) dans un échantillon biologique d'un sujet atteint d'un cancer, dans lequel lorsque au moins une cellule dans ledit échantillon présente environ dix points CEP17 ou plus, la SG et la SSP du sujet est prédite pour être inférieure à celle d'un sujet atteint d'un cancer chez qui aucune des cellules dans un échantillon biologique du sujet ne présente environ dix points CEP17 ou plus, dans lequel les cellules circulantes possèdent les caractéristiques suivantes :
(a) un grand noyau polyploïde atypique d'une taille d'environ 14-64 µm, ou plusieurs noyaux dans une seule cellule ;
(b) une taille cellulaire d'environ 20-300 µm ; et
(c) une forme morphologique sélectionnée dans le groupe consistant en une forme en fuseau, de têtard, ronde, oblongue, avec deux pieds, avec plus de deux pieds, avec des pieds fins, et amorphe.

5. Procédé selon l'une quelconque des revendications 1 à 4, dans lequel ladite SG et/ou SSP est sur une période d'au moins 12 mois, de préférence sur une période d'au moins 24 mois.

6. Procédé selon l'une quelconque des revendications 1 à 4, dans lequel la taille de l'échantillon biologique est comprise entre 5 et 15 ml.

7. Procédé selon l'une quelconque des revendications 1 à 4, dans lequel les cellules circulantes possèdent une ou plusieurs des caractéristiques supplémentaires suivantes :
(d) un phénotype positif pour CD14 ;
(e) une expression de CD45 ;
(f) une expression d'EpCAM ;
(g) une expression de la vimentine ;
(h) une expression de PD-L1 ;
(i) une expression du marqueur monocytaire CD11C ;
(j) une expression du marqueur endothélial CD146 ;
(k) une expression du marqueur endothélial CD202b ; et
(i) une expression du marqueur endothélial CD31.

8. Procédé selon l'une quelconque des revendications 1 à 4, dans lequel la source de l'échantillon biologique est un ou plusieurs parmi le sang périphérique, le sang, un ganglion lymphatique, la moelle osseuse, le liquide céphalorachidien, un tissu et l'urine.

9. Procédé selon la revendication 8, dans lequel l'échantillon biologique est du sang de la veine antécubitale, du sang de la veine cave inférieure, du sang de la veine fémorale, du sang de la veine porte, ou du sang de la veine jugulaire.

10. Procédé selon l'une quelconque des revendications 1 à 4, dans lequel le cancer est une tumeur solide, un cancer de stade I, un cancer de stade II, un cancer de stade III, un cancer de stade IV, un carcinome, un sarcome, un neuroblastome, un mélanome, le cancer des cellules épithéliales, le cancer du sein, le cancer de la prostate, le cancer du poumon, le cancer du pancréas, le cancer colorectal, le cancer du foie, le cancer de la tête et du cou, le cancer du rein, le cancer de l'ovaire, le cancer de l'oesophage ou un autre cancer à tumeur solide.

11. Procédé selon l'une quelconque des revendications 1 à 4, dans lequel les cellules circulantes sont isolées des échantillons biologiques pour les étapes de détermination en utilisant un ou plusieurs moyens sélectionnés dans le groupe consistant en une méthodologie d'exclusion stérique, une immunocapture, une lyse des globules rouges, une déplétion des globules blancs, un gradient de densité de FICOLL, l'électrophorèse, la diélectrophorèse, la cytométrie en flux, la lévitation magnétique, et diverses puces microfluidiques, ou une combinaison de ceux-ci.

12. Procédé selon la revendication 11, dans lequel les cellules circulantes sont isolées des échantillons biologiques en utilisant une méthodologie d'exclusion stérique qui comprend l'utilisation d'un microfiltre.

13. Procédé selon la revendication 12, dans lequel le microfiltre possède une taille de pore comprise entre environ 5 microns et environ 20 microns, dans lequel les pores du microfiltre possèdent une forme de pore ronde, de circuit, ovale, carrée et rectangulaire, dans lequel le microfiltre possède une géométrie de pores de précision et une distribution de pores uniforme.

14. Procédé selon la revendication 11, dans lequel les cellules circulantes sont isolées en utilisant une puce microfluidique via un tri basé sur la taille physique, un tri basé sur la taille hémodynamique, un regroupement, un piégeage, une immunocapture, une concentration des grandes cellules, ou une élimination des petites cellules en se basant sur la taille.

15. Procédé selon l'une quelconque des revendications 1 à 4, dans lequel les cellules circulantes sont isolées des échantillons biologiques pour les étapes de détermination en utilisant un essai de microfiltration à basse pression avec des microfiltres possédant plus de 160 000 pores dans un réseau uniforme ayant un diamètre de pore de 7 µm dans une surface de 9 mm.
